# EUROPEAN PATENT SPECIFICATION

(11) **EP 2 234 974 B1**
(45) Date of publication and mention of the grant of the patent: **25.04.2012**
(21) Application number: 08707040.5
(22) Date of filing: 15.01.2008
(51) Int. Cl.: C07D 211/60

(54) **PROCESS FOR THE PREPARATION OF (S)-1-ALKYL-2',6'-PIPECOLOXYLIDIDE COMPOUND**
VERFAHREN ZUR HERSTELLUNG EINER (S)-1-ALKYL-2ý,6ý-PIPECOLOXYLIDIDVERBINDUNG
PROCÉDÉ DE PRÉPARATION D'UN COMPOSÉ (S)-1-ALKYL-2',6'-PIPÉCOLOXYLIDIDE

(43) Date of publication of application: 06.10.2010
(73) Proprietor: Pharmathen S.A., 15351 Pallini Attikis (GR)
(72) Inventor: KOFTIS, Theocharis, GR-542 48 Thessaloniki (GR); SONI, Rohit, Ravikant, GR-551 34 Kalamaria (GR); GEORGOPOULOU, Ionna, GR-546 29 Anatoli A Thessaloniki (GR); LITHADIOTI, Alexandra, GR-570 01 Thermi Thessaloniki (GR)
(86) International application number: PCT/EP2008/000236
(87) International publication number: WO 2009/089841

(56) References cited:
- WO-A-85/00599
- WO-A-96/12699
- WO-A-2007/123405
- US-A- 4 870 086

## Description

### TECHNICAL FIELD OF THE INVENTION

The present invention relates to an improved process for the preparation of (S)-1-alkyl-2',6'-Pipecoloxylidide compounds and in particular to a method for the preparation of highly optically pure compounds useful as local anesthetics such as Ropivacaine and Levobupivacaine or salts thereof and pharmaceutical preparations containing said compounds.

### BACKGROUND OF THE INVENTION

N-alkyl-pipecolic acid amides are known per se and widely used as local anesthetics in the pharmaceutical industry, such as Ropivacaine or Levobupivacaine.

Ropivacaine is administered in the S-form and described in WO-A-85/00599. Levobupivacaine is the S-enantiomer of Bupivacaine and described in GB 1180712. These optically active compounds have unexpected long duration of action compared to the corresponding racemates and the R-isomers and they possess significant low cardiotoxicity. Thus, there is a need for processes to manufacture said compounds in the form of single enantiomer.

Moreover, prior art processes for the preparation of Ropivacaine or Levobupivacaine do not provide the desired yields in a high purity during the chemical reactions.

WO-A-85/00599 discloses a process for the preparation of Ropivacaine. However the preparation process disclosed in said patent has the disadvantage that it yields a product which contains about 90% of the S-isomer and relatively large amounts of the (R)-(+)-enantiomer, the undesired side product.

US-A-4870086 discloses a process for the preparation of Ropivacaine hydrochloride monohydrate with optical purity ≥ 99.5%. Further, it teaches that acetone-water purification increases the chiral purity from 90 % to ≥99.5% and that the proportion of water and acetone is very crucial to achieve the desired chiral purity.

In addition, it teaches that other solvents such as isopropanol, methanol and ethanol along with water are not suitable for crystallizing (S)-Ropivacaine hydrochloride in ≥ 99.5% optical purity.

WO-A-96/12699 discloses a resolution of 2',6'-Pipecoloxylidide and/or Bupivacaine in isopropanol and water. The impact of the water proportion in % enantiomeric excess and yield is well studied and presented in a tabular form in the same patent.

All the above prior art documents teach that enhancement of chiral purity from about 90% to ≥99.5% of (S)-2',6'-Pipecoloxylidide or its 1-alkyl analogues is achieved by crystallization in aqueous ketonic or aqueous alcoholic solvent, wherein the role of the water is a very crucial in enhancing chiral purity.

However, there is still a need to prepare these compounds with cost-effective process and a high purity of the products obtained, i.e. with minimum of expenditure on the work-up and purification of the reaction products, at higher reaction temperatures and for shorter reaction times.

### SUMMARY OF THE INVENTION

It is, therefore, an object of the present invention to provide an improved process for the preparation of essentially pure (S)-1-alkyl-2',6'-Pipecoloxylidide compound such as Ropivacaine, Levobupivacaine or salts thereof, which overcomes the deficiencies of the prior art and results to a higher chiral purity and higher yield of said compound.

Another object of the present invention is to provide a method of preparing (S)-1-alkyl-2',6'-Pipecoloxylidide compound such as Ropivacaine, Levobupivacaine or salts thereof, which results without extensive purification steps to reduction of the cost of production.

In accordance with the above objects of the present invention, a process for the preparation of (S)-1-alkyl-2',6'-Pipecoloxylidide compound such as Ropivacaine, Levobupivacaine or salts thereof, is provided comprising crystallization of (S)-1-alkyl-2',6'-Pipecoloxylidide alcanoic acid addition salt in a non-alcoholic and non-ketonic solvent.

Preferred embodiments of the present invention are set out in dependent claims 2 to 7.

Other objects and advantages of the present invention will become apparent to those skilled in the art in view of the following detailed description.

### DETAILED DESCRIPTION OF THE INVENTION

The present invention relates to an improved process for the manufacture of the compound (S)-1-alkyl-2',6'-Pipecoloxylidide or salts thereof.

According to the present invention, the process for the preparation essentially pure (S)-1-alkyl-2',6'-Pipecoloxylidide compound such as Ropivacaine, Levobupivacaine or salts thereof, comprises the following steps:
- A starting compound (S)-2',6'-pipecoloxylidide with chiral purity of about 90-92% is alkylated with alkyl halide in methyl isobutyl ketone as a solvent and potassium carbonate as a base.
- The resultant (S)-1-alkyl-2',6'-pipecoloxylide with chiral purity of about 90-92% is dissolved in a suitable non-ketonic and non-alcoholic solvent such as toluene, benzene, xylene etc. and alkanoic acid is added to crystallize as the alkanoate salt. The isolated salt has a chiral purity of ≥99.5%.
- The (S)-1-alkyl-2',6'-pipecoloxylidide acid addition salt prepared above is converted to its free base by treatment with alkali, followed by extraction with toluene, which on concentration provides the required (S)-1-alkyl-2',6'-pipecoloxylide in ≥99.5% chiral purity, which can be utilized directly for pharmaceutical preparation as a local anesthetic.

According to the present invention, (S)-1-alkyl-2',6'-Pipecoloxylidide compound such as Ropivacaine, Levobupivacaine or salts thereof, is prepared from (S)-2',6'-Pipecoloxylidide, which is used as the key starting material.

The most preferred alkanoic acids are acetic acid, propionic acid, butanoic acid and trifluoroacetic acid.

The process of the present invention will be demonstrated in more details with reference to the following examples, which are provided by way of illustration only and should not be construed as limit to the scope of the reaction in any manner.

Chiral purity in all examples of the present invention is determined by the following conditions:
100mm x 40mm Chiral AGP column; λₘₐₓ 220nm; mobile phase prepared by diluting 35 ml of isopropanol buffer pH 7.2 up to volume 500 ml; buffer preparation: 7.5 ml NaH₂PO₄ 1M + 28.5 ml Na₂HPO₄ 0.5M diluted to 1 liter of water adjusted to pH 7.2; injection volume 20 µl; sample and standard preparation carried out in concentration 75ppm.

### Example 1: Preparation of (S)-1-Propyl-2',6'-pipecoloxylidide (Ropivacaine)

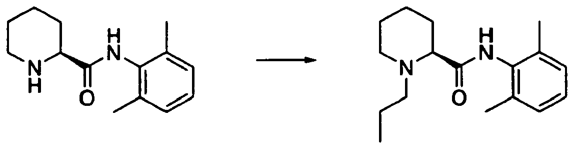

In a round bottom flask equipped with a condenser and a magnetic stirrer, 10.0 g of (*S*)-2',6'-pipecoloxylidide(with chiral purity ~90-92%) was dissolved in 40 ml of methyl isobutyl ketone, 5.9 g K₂CO₃ and 3.9 ml water were added. Then 11.4 ml of 1-bromopropane was added to the reaction mixture and said reaction mixture was heated to 85-90°C and maintained for 3-4 hours under vigorous stirring until completion of the reaction. The solvents were removed in a rotary evaporator, the reaction mass was quenched with 120 ml water and extracted first with 100 ml toluene, followed by two more extractions with 50 ml of toluene. The three organic extracts were combined, dried over MgSO₄, the solids were filtered off, 3.7 ml of glacial acetic acid was added and the reaction mass was stirred for 10-15 minutes. The solvent was removed in a rotary evaporator and the crude mass was dissolved in 60 ml of toluene under reflux and allowed to cool to 20°C, which after filtration provided 10.7g of (S)-1-Propyl-2',6'-Pipecoloxylidide acetate salt with 99.9 % chromatographic purity and 99.67 % chiral purity. The free base was generated by dissolving the acetate salt in 120 ml water, followed by treatment with 7 ml of 50 % sodium hydroxide solution and three extractions (100 ml+50 ml+50ml) with toluene. The three organic extracts were combined and concentrated to provide 8.2 g. of (S)-1-Propyl-2',6'-pipecoloxylidide.

### Example 2: Preparation of (S)-1-Butyl-2',6'-pipecoloxylidide (Levobupivacaine)

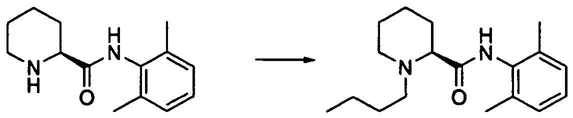

In a round bottom flask equipped with condenser and magnetic stirrer, 4.4 g of (*S*)-2',6'-pipecoloxylidide (with chiral purity ~90-92%) was dissolved in 22 ml of methyl isobutyl ketone, 2.6 g K₂CO₃ and 2.2 ml water added. Then 3.2 ml 1-Iodobutane was added to the above mixture and said reaction mixture was heated to 85-90°C for 3.5 hours under vigorously stirring until completion of the reaction. the reaction mass was quenched with 60 ml water and extracted first with 50 ml toluene, followed by two more extractions with 25 ml of toluene. The three organic extracts combined, dried with Na₂SO₄, the solids filtered off and 0.76 ml glacial acetic acid added. The solvents removed in a rotary evaporator and the crude mass dissolved in 11 ml toluene under reflux and let to cool down to 20°C that after filtration provided 2.8 g of (*S*)-1-Butyl-2',6'-pipecoloxylidide acetate salt. This suspended in 30 ml toluene and 50 ml water and 4 ml of aq.sodium hydroxide (50% w/w) added and the mixture stirred vigorously for 10 min. The mixture transferred to a separatory funnel. The organic layer separated and the aqueous layer extracted twice with 25 ml of toluene. The three organic extracts combined, dried with Na₂SO₄, the solids filtered off and the filtrate on concentration on rotary evaporator followed by cooling provided 2.1g. (*S*)-1-butyl-2',6'-pipecoloxylidide with S-isomer 99.64% chiral purity

### Example 3: Preparation of (S)-1-Methyl-2',6'-pipecoloxylidide (S-Mepavacaine)

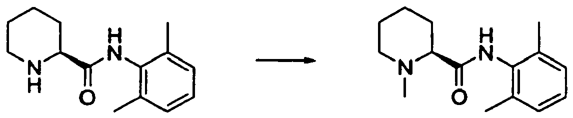

In a round bottom flask equipped with condenser and magnetic stirrer, 4.4 g of (*S*)-2', 6'-pipecoloxylidide (with chiral purity ~90-92%) was dissolved in 22 ml of methyl isobutyl ketone, 2.6 g K₂CO₃ and 2.2 ml water added. Then 1.74 ml Methyl iodide was added to the mixture and the reaction mixture was heated to 85-90°C for 4 hours under vigorously stirring until completion of the reaction, the reaction mass was quenched with 60 ml water and extracted first with 50 ml toluene, followed by two more extractions with 25 ml of toluene. The three organic extracts combined, dried with Na₂SO₄, the solids filtered off and 0.76 ml glacial acetic acid added. The solvents removed in a rotary evaporator and the crude mass dissolved in 11 ml toluene under reflux and let to cool down to 20°C that after filtration provided 2.0 g of (*S*)-1-Methyl-2',6-pipecoloxylidide acetate salt. This suspended in 30 ml toluene and 50 ml water and 4 ml of aqueous sodium hydroxide solution (50% w/w) added and the mixture stirred vigorously for 10 minutes. The mixture transferred to a separatory funnel, the organic layer separated and the aqueous layer extracted twice with 25 ml of toluene. The three organic extracts combined, dried with Na₂SO₄, the solids filtered off and the filtrate on concentration on rotary evaporator followed by cooling provided 1.5 g (*S*)-1-Methyl-2',6'-pipecoloxylidide with chiral purity of 99.7% .

### Example 4: Preparation of (S)-1-Ethyl-2',6'-pipecoloxylidide

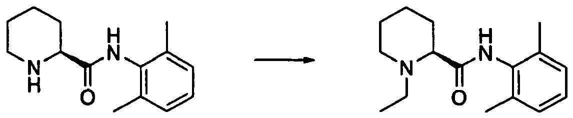

In a round bottom flask equipped with condenser and magnetic stirrer, 4.4 g of (*S*)-2',6'-pipecoloxylidide (with chiral purity ~90-92%) was dissolved in 22 ml of methyl isobutyl ketone, 2.6 g K₂CO₃ (1.0 equiv) and 2.2 ml water added. Then 4.2 ml Ethyl bromide was added to the mixture and said reaction mixture was heated to 85-90 °C for 3.5 hours under vigorously stirring until completion of the reaction, the reaction mass was quenched with 60 ml water and extracted first with 50 ml toluene, followed by two more extractions with 25 ml of toluene. The three organic extracts combined, dried with Na₂SO₄, the solids filtered off and 1.85 ml glacial acetic acid added. The solvents removed in a rotary evaporator to get the crude mass(4.2 g.), which is dissolved in 22 ml toluene under reflux and allowed to cool down to 20°C that after filtration provided 3.0 g of (*S*)-1-Ethyl-2',6'-pipecoloxylidide acetate salt. This dissolved in 60 ml of water, basified with 50% sodium hydroxide solution(~5 ml) and extracted with toluene (50 ml + 25 ml + 25 ml) toluene The three organic extracts combined, dried with Na₂SO₄, the solids filtered off and the solvents removed in a rotary evaporator to provide 1.5g of (*S*)-1-Ethyl-2',6'-pipecoloxylidide with 99.8 % chiral purity.

The present invention provides a process wherein chiral purity of different derivatives of (S)-1-alkyl-2',6'-Pipecoloxylidide and (S)-2',6'-pipecolxylidide is increased from about 90% to ≥99.5% in a non-ketonic and non-alcoholic solvent system, such as toluene, benzene, xylene etc. under non aqueous conditions in high yield.

According to the present invention, the respective analogues of (S)-1-alkyl-2',6'-Pipecoloxylidide with chiral purity of about 90-92% are converted to their acid addition salts with aliphatic alkanoic acid, which on crystallization in a suitable solvent provides a product with chiral purity at least 99.5%.

Therefore, the present invention describes a method of preparing essentially pure (S)-1-alkyl-2',6'-Pipecoloxylidide compound such as Ropivacaine, Levobupivacaine or salts thereof in an improved manner.

While the present invention has been described with respect to the particular embodiments, it will be apparent to those skilled in the art that various changes and modifications may be made in the invention without departing from the spirit and scope thereof, as defined in the appended claims.

## Claims

1. A process for the preparation of high chiral purity (S)-1-alkyl-2',6'-Pipecoloxylidide compound such as Ropivacaine, Levobupivacaine or salts thereof, which comprises crystallization of (S)-1-alkyl-2',6'-Pipecoloxylidide acid addition salt in a non-alcoholic and non-ketonic solvent, wherein said acid addition salt is an alkanoic acid addition salt.

2. The process according to claim 2, wherein said alkanoic acid addition salt is selected from a group consisting of the acetate, propanoate, butanoate and trifluoroacetate addition salt.

3. The process according to claim 1, wherein said chiral purity is at least 99.5%

4. The process according to claim 1, wherein the solvents used for the crystallization are hydrocarbons.

5. The process according to claim 5, wherein said hydrocarbons used for the crystallization are aromatic hydrocarbons.

6. The process according to claim 6, wherein the aromatic hydrocarbon used for the crystallization is toluene.

7. The process according to claim 1, wherein said crystallization is performed in non-aqueous medium.

## Patentansprüche

1. Ein Verfahren zur Herstellung von hoch chirale Reinheit (S)-1-alkyl-2',6'-Pipecoloxylidide wie Ropivacain, Levobupivacain oder deren Salze, enthaltend die Kristallisation von (S)-1-alkyl-2',6 '-Pipecoloxylidide Säureadditionssalze in einer nicht-alkoholische und nicht-Keton Lösungsmittel, wobei die Säurezugabe ein alkanoid Säureadditionssalz ist.

2. Das Verfahren gemäß Anspruch 1, wobei alkanoid Säureadditionssalz aus einer Gruppe bestehend aus Acetat, propanoat, butanoat und Trifluoracetat Additionssalz ausgewählt ist.

3. Das Verfahren gemäß Anspruch 1, wobei die chirale Reinheit von mindestens 99,5% ist.

4. Das Verfahren nach Anspruch 1, wobei das Lösungsmittel für die Kristallisation, Kohlenwasserstoffe verwendet werden.

5. Das Verfahren gemäß Anspruch 4, **dadurch gekennzeichnet, dass** Kohlenwasserstoffe für die Kristallization, aromatische Kohlenwasserstoffe eingesetzt werden.

6. Das Verfahren nach Anspruch 5, wobei der aromatische Kohlenwasserstoff für die Kristallisation, Toluol verwendet wird.

7. Das Verfahren gemäß Anspruch 1, wobei die Kristallisation in nicht-wässerigen Medium durchgeführt wird.

## Revendications

1. Un procédé pour la préparation des composés de (S)-1-alkyl-2',6'-pipécoloxylidide, comme la ropivacaïne, la lévobupivacaïne ou leur sels, de haute pureté optique comprenant la cristallisation du sel d'addition acide du (S)-1-alkyl-2',6'-pipecoloxylidide dans un solvant non-alcoolisé et non-cétonique, dans lequel le dit sel d'addition d'acide est un sel d'acide alcanoïque.

2. Le procédé selon la revendication 1, dans lequel le dit sel d'addition d'acide alcanoïque est sélectionné parmi le groupe formé par les sels d'addition d'acétate, de propanoate, de butanoate et de trifluoroacétate.

3. Le procédé selon la revendication 1, dans lequel la pureté optique est d'au moins de 99.5%;

4. Le procédé selon la revendication 1, dans lesquels les solvants utilisés pour la cristallisation sont des hydrocarbures.

5. Le procédé selon la revendication 4, dans lesquels les dits hydrocarbures utilisés pour la cristallisation sont des hydrocarbures aromatiques.

6. Le procédé selon la revendication 5, dans lequel l'hydrocarbure aromatique utilisé pour la cristallisation est le toluène.

7. Le procédé selon la revendication 1, dans lequel la dite cristallisation est réalisée en milieu non-aqueux.
